# EUROPEAN PATENT SPECIFICATION

(11) **EP 0 653 938 B1**
(45) Date of publication and mention of the grant of the patent: **26.08.1998**
(21) Application number: 94906788.8
(22) Date of filing: 05.08.1993
(51) Int. Cl.: A61K 33/30, A61L 15/44, A61K 9/06

(54) **TOPICAL COMPOSITION**
TOPISCHES MITTEL
COMPOSITION A USAGE LOCAL

(30) Priority: 08.08.1992 GB 9216879
(43) Date of publication of application: 24.05.1995
(73) Proprietor: Seton Healthcare Group plc, Oldham Lancashire OL1 3HS (GB)
(72) Inventor: NESBIT, Michael, Robert 168 Eastgate, Lincolnshire LN11 9AB (GB)
(74) Representative: Goodwin, Mark
(86) International application number: GB9301662
(87) International publication number: WO9403190

(56) References cited:
- EP-A- 0 040 378
- CH-A- 667 010
- DE-C- 384 135
- FR-A- 2 083 366
- FR-A- 2 649 318
- DATABASE WPI Derwent Publications Ltd., London, GB; AN 82-03408J & JP,A,57 171 907 (KANEBO) 22 October 1982
- DATABASE WPI Derwent Publications Ltd., London, GB; AN 91-291703 & JP,A,3 193 710 (KANEBO KK) 23 August 1991

## Description

This invention relates to a topical composition comprising zinc oxide particularly, but not exclusively for impregnating a bandage.

Bandages impregnated with pastes containing zinc oxide are commonly used for the treatment of skin complaints such as leg ulcers, varicose eczemas and chronic determatitis. These known pastes are not sufficiently emulsified and are therefore unstable, particularly when subjected to sterilization processes such as heating and irradiation. As these known pastes cannot be properly sterilized it is conventional to include "preservatives" which include antibacterial agents, such as alkyl p-hydroxybenzoates, in order to irradicate any bacteria that are not eliminated by other sterilization techniques. These preservatives cause hyposensitivity when the bandage comprising the paste is applied to the wound.

EP-A-0040378 discloses sterilizable mixtures containing zinc oxide, celluloseether, glycerin, water and up to 5% of further additives.

FR-A-2649318 discloses pre-emulsions for dermatological or cosmetic use, which may comprise zinc oxide and which do not comprise a preservative agent.

The present invention seeks to provide alternative sterilization stable topical compositions.

According to the present invention there is provided a topical composition for impregnating a bandage comprising zinc oxide in a stable emulsion comprising from 0.2 to 16% wt of at least one fat and/or oil, at least one emulsifying agent, at least one inorganic suspending agent, xanthan gum and water, in the absence of a preservative.

The stable emulsions of the compositions of the present invention eliminate the need for a preservative. Thus topical zinc oxide containing compositions can be made which do not suffer from the drawback of sensitivity.

The compositions of the invention are primarily intended for impregnating bandages for use in the treatment of skin diseases.

The zinc oxide is the active agent. The composition preferably comprises from 0.5% to 40.0% wt of zinc oxide and more preferably substantially 15% wt of zinc oxide. The composition may comprise additional active ingredients such as calamine, icthammol, clioquinol and coal tar. The composition preferably comprises from 0 to 40% wt of additional actives.

In a preferred embodiment of the invention the fats and/or oils comprise fractionated coconut oil and/or synthetic spermacetti, combinations of cetyl alcohol; stearyl alcohol; other alcohols (C₈ to C₂₄); triglyceride oils or fats; esters such as cetyl palmitate, myristyl myristate; liquid and solid paraffins or other commonly used cosmetic fats and oils. The fats and/or oil form an oil in water emulsion.

The emulsifying agent is preferably polyethoxylated. Preferred examples include tween 80 polysorbate 20, 21, 40, 60, 61, 65, 81, 85, 120 and other polyoxyethylene adducts of sorbitan esters; fatty acids; fatty alcohols; lanolin; lanolin alcohols; castor oil (natural or hydrogenated) or alkylbenzenes. The emulsifying agent has a high HLB and emulsifies the fat and/or oils into the oil in water emulsion. The composition preferably comprises from 0.3 to 10% wt and still more preferably from 0.5 to 2% wt of emulsifying agent.

The water soluble viscosity improver increases the viscosity of the aqueous phase for stabilisation. One preferred material is xanthan gum. The composition preferably comprises from 0.05% to 4.0% of viscosity improver and more preferably from 0.05 to 1.2%.

In a preferred embodiment of the invention the compositions preferably comprise the following ingredients either alone or in combination: at least one low HLB emulsifying agent to emulsify the fats/and or oils and glycerol which acts as a humectant in order to improve the water retention of the composition and to provide emollient properties to the skin.

In order that the present invention may be more readily understood a specific embodiment thereof will now be described with reference to the following example:-

### EXAMPLE 1

A topical composition in accordance with the present invention was made according to the following formula:

| | % w/w |
|---|---|
| Purified Water Ph Eur | 49.9 |
| Glycerol Ph Eur | 30.0 |
| Zinc Oxide Ph Eur | 15.0 |
| Fractionated Coconut Oil (Miglyol 812) | 2.0 |
| Aluminium Magnesium Silicate BP (Veegum F) | 1.0 |
| Xanthan Gum USNF (Keltrol F) | 0.8 |
| Polysorbate 80 Ph Eur (Tween 80) | 0.5 |
| Synthetic Spermacetti USNF (Crodamol SS) | 0.5 |
| Sorbitan Mono-oleate BP (Span 80) | 0.3 |

The said formula of Example 1 was made in the following manner:

| Part A | Per 100 Kg of Batch |
|---|---|
| Purified Water Ph Eur | 49.9 Kg |
| Veegum F | 1.0 Kg |
| Tween 80 | 500 g |
| Glycerol Ph Eur | 30 Kg |
| Zinc Oxide | 15 Kg |
| Keltrol F | 800 Kg |

| Part B | |
|---|---|
| Miglyol 812 | 2.0 Kg |
| Crodamol SS | 500 g |
| Span 80 | 300 g |

The Veegum F was added to the water of the Part A Mixture and mixed with high shear agitation, whilst heating to 60°C. The other ingredients of Part A were then added and mixed with high shear agitation at 60°C. The Part B mixture was mixed in a separate small vessel and heated to 60°C. A 10 litre portion of Part A was slowly added to the Part B mixture with brisk stirring. The Part A/B mixture was then added to the remainder of the Part A mixture and mixed with agitation. The vessel used for the A/B mixture was rinsed out with portions of the batch. The batch was periodically mixed with agitation whilst cooling, until the viscosity increased and the batch was then cooled to room temperature with periodic stirring, prior to assembly.

The composition of Example 1 was stable when subjected to 120°C for 15 minutes. The composition was also stable when subjected to gamma irradiation.

The formulation of Example 1 therefore provides a stable zinc oxide containing emulsion which may be subjected to conventional sterilization techniques.

It is to be understood that the example described above is by way of illustration only. Many modifications and variations are possible.

## Claims

1. A topical composition for impregnating a bandage comprising zinc oxide in a stable emulsion comprising from 0.2 to 16% wt of at least one fat and/or oil, at least one emulsifying agent, at least one inorganic suspending agent, xanthan gum and water, in the absence of a preservative.

2. A composition as claimed in claim 1, wherein the composition comprises from 0.5% to 40.0% wt of zinc oxide.

3. A composition as claimed in claim 1 or claim 2, wherein the composition comprises one or more further additional active ingredients including at least one of the following: calamine, icthammol, clioquinol or coal tar.

4. A composition as claimed in claim 3, wherein the composition comprises up to 40% wt of said at least one additional active ingredient.

5. A composition as claimed in any preceding claim, wherein the fat and/or oil comprises any of the following: fractionated coconut oil and/or synthetic spermacetti, combinations of cetyl alcohol; stearyl alcohol; other alcohols (C₈ to C₂₄); triglyceride oils or fats; esters such as cetyl palmitate, myristyl myristate; liquid and solid paraffins.

6. A composition as claimed in any preceding claim, wherein the emulsifying agent comprises a polyethyoxylated emulsifying agent.

7. A composition as claimed in any preceding claim, wherein the emulsifying agent includes any of the following: tween 80 polysorbate 20, 21, 40, 60, 61, 65, 81, 85, 120 or other polyoxyethylene adducts of sorbitan esters; fatty acids; fatty alcohols; lanolin; lanolin alkylbenzenes.

8. A composition as claimed in any preceding claim, wherein the composition comprises from 0.3 to 10% wt of emulsifying agent.

9. A composition as claimed in any preceding claim, wherein the composition comprises from 0.5 to 2% wt of emulsifying agent.

10. A composition as claimed in any preceding claim, wherein the composition comprises from 0.05% to 4.0% wt of xanthan gum.

11. A composition as claimed in any preceding claim, wherein the composition comprises from 0.05% to 1.2% wt of xanthan gum.

12. A composition as claimed in any preceding claim, wherein the composition comprises at least one emulsifying agent for emulsifying the fats and/or oils.

13. A composition as claimed in any preceding claim, wherein the composition further comprises glycerol.

14. A bandage impregnated by the composition of any of claims 1 to 13.

## Patentansprüche

1. Topische Zusammensetzung zum Tränken von Verbänden, die Zinkoxid in Form einer beständigen Emulsion aufweist, welche 0,2 bis 16 Gew.% mindestens eines Fettes und/oder Öls, sowie mindestens einen Emulgator, mindestens ein anorganisches Suspensionsmittel, Xanthan und Wasser, aber kein Konservierungsmittel enthält.

2. Zusammensetzung nach Anspruch 1, dadurch gekennzeichnet, daß sie 0,5 bis 40,0 Gew.% Zinkoxid enthält.

3. Zusammensetzung nach Anspruch 1 oder 2, dadurch gekennzeichnet, daß sie einen oder mehrere zusätzliche Wirkstoffe enthält, zu denen mindestens einer der folgenden zählt: Kalamin, Ichthammol, Clioquinol oder Kohlenteer.

4. Zusammensetzung nach Anspruch 3, dadurch gekennzeichnet, daß sie bis zu 40 Gew.% des genannten, mindestens einen, zusätzlichen Wirkstoffs enthält.

5. Zusammensetzung nach einem oder mehreren der vorherigen Ansprüche, dadurch gekennzeichnet, daß das Fett und/oder Öl jeden beliebigen der folgenden Stoffe umfaßt: fraktioniertes Kokosöl und/oder synthetisches Spermacet, Kombinationen von Cetylalkohol, Stearylalkohol, sonstigen Alkoholen (C₈ bis C₂₄), Triglyceridölen oder -fetten, Estern wie z.B. Palmitinsäurecetylester, Myristylmyristat; flüssigen und festen Paraffinen.

6. Zusammensetzung nach einem oder mehreren der vorherigen Ansprüche, dadurch gekennzeichnet, daß der Emulgator polyethoxyliert ist.

7. Zusammensetzung nach einem oder mehreren der vorherigen Ansprüche, dadurch gekennzeichnet, daß der Emulgator jeden beliebigen der folgenden Stoffe umfaßt: Tween 80, Polysorbat 20, 21, 40, 60, 61, 65, 81, 85, 120 oder andere Polyoxyethylenaddukte von Sorbitanestern, Fettsäuren, Fettalkoholen, Lanolin, Lanolinalkylbenzolen.

8. Zusammensetzung nach einem oder mehreren der vorherigen Ansprüche, dadurch gekennzeichnet, daß sie 0,3 bis 10 Gew.% Emulgator enthält.

9. Zusammensetzung nach einem oder mehreren der vorherigen Ansprüche, dadurch gekennzeichnet, daß sie 0,5 bis 2 Gew.% Emulgator enthält.

10. Zusammensetzung nach einem oder mehreren der vorherigen Ansprüche, dadurch gekennzeichnet, daß sie 0,05 bis 4,0 Gew.% Xanthan enthält.

11. Zusammensetzung nach einem oder mehreren der vorherigen Ansprüche, dadurch gekennzeichnet, daß sie 0,05 bis 1,2 Gew.% Xanthan enthält.

12. Zusammensetzung nach einem oder mehreren der vorherigen Ansprüche, dadurch gekennzeichnet, daß sie mindestens einen Emulgator zum Emulgieren der Fette und/oder Öle enthält.

13. Zusammensetzung nach einem oder mehreren der vorherigen Ansprüche, dadurch gekennzeichnet, daß sie außerdem Glycerin enthält.

14. Mit der Zusammensetzung nach einem oder mehreren der Ansprüche 1 bis 13 getränkter Verband.

## Revendications

1. Composition topique pour l'imprégnation d'un bandage comprenant de l'oxyde de zinc dans une émulsion stable comprenant de 0,2 à 16% en poids d'au moins une graisse et/ou huile, d'au moins un agent émulsifiant, d'au moins un agent de suspension minérale, de la gomme de xanthane et de l'eau, en l'absence d'agent conservateur.

2. Composition selon la revendication 1, dans laquelle la composition comprend de 0,5 à 40,0% en poids d'oxyde de zinc.

3. Composition selon la revendication 1 ou 2, dans laquelle la composition comprend une ou plusieurs autres substances actives comprenant au moins l'une parmi les suivantes : de la calamine, de l'ichtammol, du clioquinol ou du groudron de houille.

4. Composition selon la revendication 3, dans laquelle la composition comprend jusqu'à 40% en poids d'au moins une substance active additionnelle.

5. Composition selon l'une quelconque des revendications précédentes, dans laquelle la graisse et/ou l'huile comprend l'un quelconque des constituants suivants : de l'huile de copra fractionnée et/ou de l'huile de baleine synthétique, des combinaisons d'alcool cétylique; de l'alcool stéarylique; d'autres alcools en C₈ à C₂₄; des huiles ou graisses de type triglycéride; des esters tels que le palmitate de cétyle, le myristate de myristyle; des paraffines liquides et solides.

6. Composition selon l'une quelconque des revendications précédentes, dans laquelle l'agent émulsifiant comprend un agent émulsifiant polyéthoxylé.

7. Composition selon l'une quelconque des revendications précédentes, dans laquelle l'agent émulsifiant comprend l'un quelconque des constituants suivants : du tween 80, du polysorbate 20, 21, 40, 60, 61, 65, 81, 85, 120 ou d'autres adduits de polyoxyéthylène avec des esters de sorbitanne; des acides gras; des alcools gras; de la lanoline; des lanolinealkylbenzènes.

8. Composition selon l'une quelconque des revendications précédentes, dans laquelle la composition comprend de 0,3 à 10% en poids d'un agent émulsifiant.

9. Composition selon l'une quelconque des revendications précédentes, dans laquelle la composition comprend de 0,5 à 2% en poids d'un agent émusifiant.

10. Composition selon l'une quelconque des revendications précédentes, dans laquelle la composition comprend de 0,05 à 4,0% en poids de gomme de xanthane.

11. Composition selon l'une quelconque des revendications précédentes, dans laquelle la composition comprend de 0,5 à 1,2% en poids de gomme de xanthane.

12. Composition selon l'une quelconque des revendications précédentes, dans laquelle la composition comprend au moins un agent émulsifiant pour l'émulsification des graisses et/ou huiles.

13. Composition selon l'une quelconque des revendications précédentes, dans laquelle la composition comprend en outre du glycérol.

14. Bandage imprégné par une composition selon l'une quelconque des revendications 1 à 13.
